# EUROPEAN PATENT SPECIFICATION

(11) **EP 0 576 604 B1**
(45) Date of publication and mention of the grant of the patent: **12.01.2000**
(21) Application number: 92910284.6
(22) Date of filing: 20.03.1992
(51) Int. Cl.: A61M 5/152

(54) **CONSTANT-PRESSURE FLUID SUPPLY SYSTEM WITH MULTIPLE FLUID CAPABILITY**
GLEICHDRUCKFLUIDZUFUHRSYSTEM MIT MEHRFLÜSSIGKEITSFÄHIGKEIT
SYSTEME D'ALIMENTATION EN FLUIDES MULTIPLES SOUS UNE PRESSION CONSTANTE

(30) Priority: 22.03.1991 US 673835; 22.08.1991 US 748346
(43) Date of publication of application: 05.01.1994
(73) Proprietor: DEKA PRODUCTS LIMITED PARTNERSHIP, Manchester, NH 03101 (US)
(72) Inventor: KAMEN, Dean, L., Bedford, NH 03102 (US); NORMAND, Gerard, B., Manchester, NH 03102 (US); DURAND, Kevin, Lancaster, MA 01523 (US); SEALE, Joseph, B., Gorham, ME 04038 (US)
(74) Representative: Billington, Lawrence Emlyn
(86) International application number: US9202291
(87) International publication number: WO9216304

(56) References cited:
- EP-A- 0 380 862
- WO-A-88/03819
- DE-A- 2 731 448
- DE-A- 3 605 664
- US-A- 3 506 005
- US-A- 3 677 444

## Description

The present invention relates generally to systems for supplying and controlling fluid flow, and in particular to medical infusion technology, although other embodiments are possible.

The invention provides a system for dispensing fluid at relatively constant pressure. An infusion pump for dispensing a fluid at constant pressure is known from WO 88/03819. However, this is capable of administering a single fluid only. WO-A-90/13795 describes a device comprising two administering chambers.

According to the present invention there is provided a fluid dispensing system comprising: a resilient membrane; and means for mounting the resilient membrane so as to provide a pressure chamber of variable dimension which constitutes or includes a first dispensable fluid chamber of variable dimension having a fluid conduit, the system including a second dispensable fluid chamber of variable dimension having a fluid conduit; an output manifold; and switch means for serially switching the conduits from the dispensable fluid chambers into the output manifold so as to enable first and second fluids to be sequentially dispensed, characterised in that the second fluid chamber of variable dimension is disposed within the pressure chamber.

In an embodiment, the pressure chamber is the first dispensable fluid chamber and the resilient member defining it is a pre-tensioned resilient membrane. In this case, the second dispensable fluid chamber may be substantially surrounded by the first dispensable fluid chamber. In another embodiment, the fluid dispensing chambers are substantially surrounded by another fluid contained within the pressure chamber of variable dimension defined by a pre-tensioned resilient membrane.

In a preferred embodiment, the resilient membrane is pre-tensioned beyond the point at which, as fluid enters the chamber defined by the membrane and the volume of the chamber increases, the first derivative of pressure over volume becomes zero for the first time.

The invention will be more readily understood by reference to the following description taken with the accompanying drawings in which:
Figs. 1 and 2 illustrate the characteristics of a resilient membrane of the type used in accordance with the invention;
Fig. 3 shows a cross section through a fluid dispensing system for illustrative purposes;
Fig. 4 is a cross section through an embodiment of a system of the invention having the capability of supplying sequentially a series of fluids in any desired order;
Fig. 5 is a schematic plan view of another embodiment of a system of the invention;
Fig. 6 is a schematic elevation view of the embodiment of Fig. 5;
Fig. 7 is a schematic cross-section view of the digital valves of the embodiment of Fig. 5; and
Fig. 8 illustrates another fluid dispensing system for illustrative purposes.

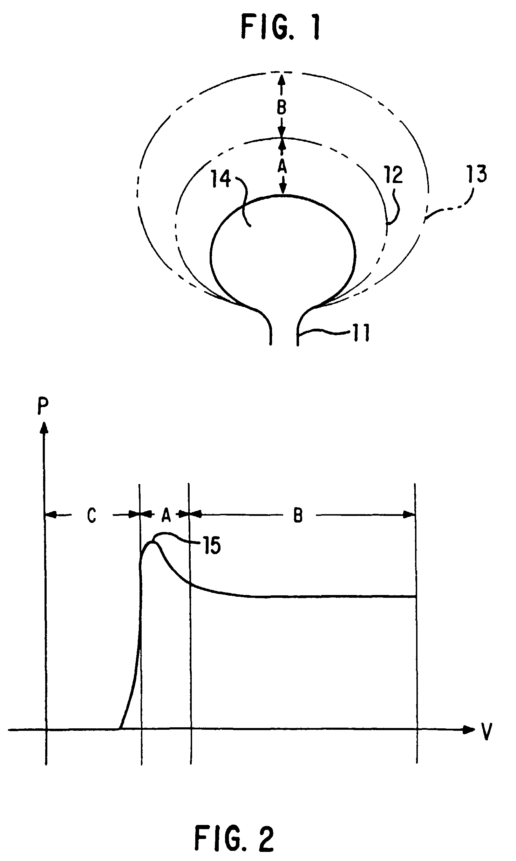

Fig. 1 illustrates the use of a resilient membrane 11 of the type suitable for the system of the present invention. A membrane of substantially uniform thickness when filled to a requisite degree with fluid will tend to assume a spherical shape. Although any initial shape is possible, the membrane shown here is assumed to be substantially spherical in shape prior to any substantial amount of stretching caused by the introduction of fluid into the interior volume 14 of the chamber defined by the membrane. As additional fluid is introduced into the membrane, its shape grows larger and reaches extents indicated by 12 and thereafter 13 in Fig. 1. Until fluid has substantially filled the volume 14 of membrane 11 (or more precisely until the pressure inside the volume 14 exceeds ambient pressure), the membrane is not stretched. However, at some point fluid cannot be introduced into volume 14 without causing stretching of the walls of the membrane 11 and a consequent increase in pressure in the volume 14 within the membrane.

Fig. 2 illustrates the pressure versus volume characteristics of the resilient membrane shown in Fig. 1.

To achieve initial growth in volume--in the region identified as region A in Figs. 1 and 2--a comparatively large pressure is required to initiate the stretching of the membrane that is necessary to increase the volume 14 within it. Once the initial stretching has occurred, however, over a substantial volume range indicated by region B in Figs. 1 and 2, the pressure tends to remain substantially constant. There is a point at which, as fluid enters the volume contained by the resilient membrane and the volume increases, the first derivative of pressure over volume becomes zero for the first time (15 in Fig. 2). At some point, of course, the membrane will enter a condition under which it has been stretched beyond its ability to maintain these characteristics and is subject to breakage. Region B is defined not to extend to that condition.

Typically delivery systems of the general type to which the invention is applicable must cope with the characteristics described in Figs. 1 and 2. They typically operate in regions A and B in the course of delivery of intravenous fluids. In fact, near the end of the delivery cycle, as the volume 14 of membrane 11 reaches its initial size, the pressure-volume relationship is in fact in the region identified as C in Fig. 2, and the pressure drops precipitously to zero before all the fluid has been delivered, leaving some fluid still remaining in the device.

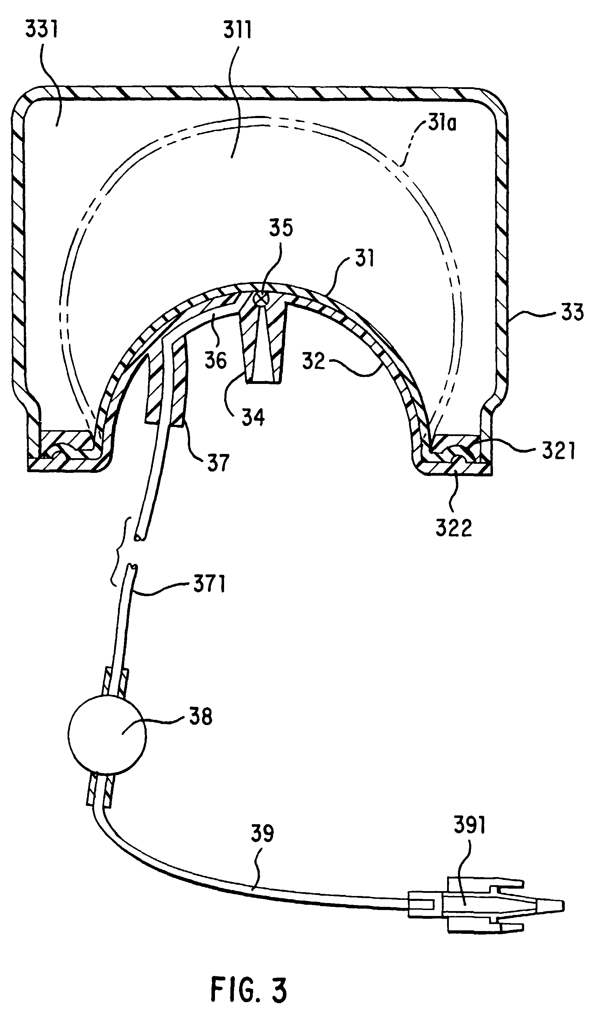

The present invention solves the problems posed in delivery systems of this type by providing an arrangement for mounting a resilient membrane under tension in such a way that the membrane operates only in the region B of Fig. 2. By way of explanation, in the system shown in Fig. 3, there is provided a mandrel to cause the membrane to occupy a shape, when the membrane is fully contracted and the volume of the chamber defined by the membrane is substantially at zero, that the membrane would otherwise occupy when the system is filled with fluid to the point that the membrane first reaches the constant pressure mode. In this system, the interior volume is substantially zero when the system is devoid of fluid. In Fig. 3 membrane 31 is shown pre-tensioned, fully contracted and stretched over mandrel 32 so that the volume of the chamber defined between the membrane and the mandrel is substantially zero. In Fig. 3, membrane 31 is supported in this initial (zero volume) position by mandrel 32 in substantially the same generally hemispherical shape that it would have occupied had it been filled with fluid as shown in Fig. 1 to a volume 12, corresponding to the lower end of range B in Fig. 2.

The membrane 31 is thus mounted under tension around mandrel 32 and sandwiched between toroidal cap 321 and the foot 322 of the mandrel. In a typical preferred embodiment, the membrane provides a pressure of about 35 kPa (about five pounds per square inch) over its operating range. When the membrane is nearly fully expanded, it occupies the position identified as 31a, and defines a chamber of interior volume 311. Case 33 provides a maximum limit on stretching of the membrane 31 and consequently on the size of volume 311.

The volume 331 between the membrane 31 and the case 33 is desirably vented to atmosphere in many applications to permit expansion and contraction of the membrane without compression and decompression of air in the volume 331. Venting may be achieved by any suitable means, including an orifice in case 33 or the creation of one or more channels between cap 321 and case 33; such channels could, for example, extend additionally between the foot 322 of mandrel 32 and the proximate portion of the case 33.

Initial filling of the system is achieved via luer fitting 34 and one-way valve 35. One suitable arrangement for implementing items 34 and 35 is disclosed in United States patent number 5116021 filed August 22, 1991, entitled "Quick-Disconnect Valve," for an invention of Dean Kamen and Valentine Faust. That patent is hereby incorporated herein by reference.

Fluid is dispensed by the system through channel 36 and fitting 37, which are integrally formed with mandrel 32. From the fitting 37, the fluid flow is through line 371, filter 38, and flow-restrictive tubing 39, and through output fitting 391, which may be a luer fitting that is suitably capped until use of the system. The flow-restrictive tubing 39 has a specific and small cannula size so as to help assure constant pressure at the fitting 391 and provide a specified maximum flow rate through the fitting 391. The filter 38 provides filtering of the fluid and may also provide air elimination in manners known in the art. The line 371 may be clamped or otherwise modified to control flow in a manner known in the art. Alternatively, for example, flow can be controlled in a manner described in United States patents number 5088515 and number 4,976,162.

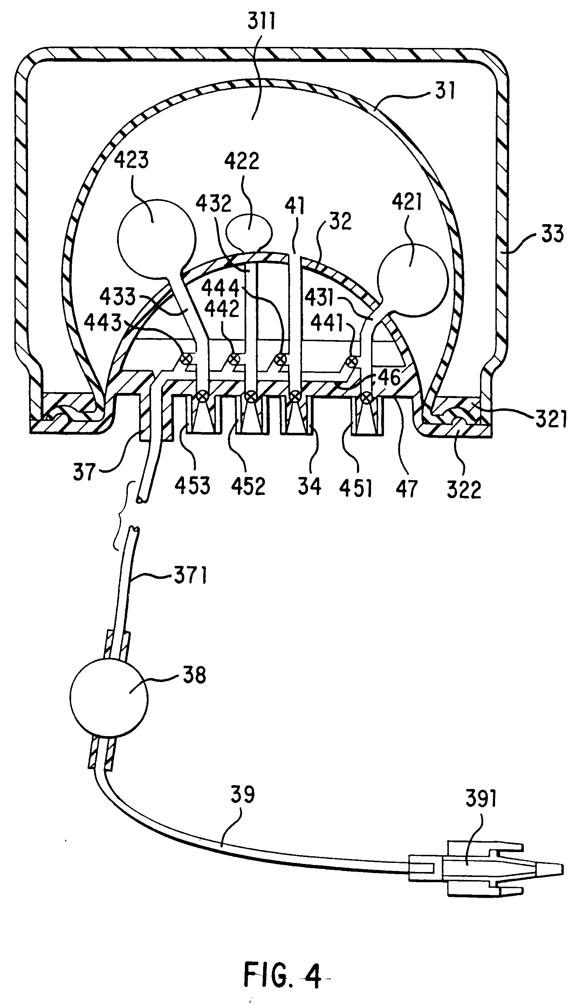

Fig. 4 shows a system similar to that shown in Fig. 3 but capable of dispensing automatically in sequence a series of fluids in any desired order in accordance with the present invention. In this embodiment, one fluid is placed in volume 311 of the dispensing chamber defined by main membrane 31 and other fluids are placed in volumes 421, 422 and 423 of three additional dispensing chambers define by three additional membranes, which are disposed in the volume 311 between the main membrane 31 and the mandrel 32. The actual number of additional membranes is a matter of design choice, and here the three additional membranes are spaced at equal angles around the mandrel 32. The additional membranes are slack, i.e., they are not used in a stretched condition. Therefore, the pressure exerted by the stretching of the main membrane 31 of the main interior volume 311 is exerted equally on each additional interior volume defined by each additional (slack) membrane. Fluid may be dispensed from main volume 311 and from each additional volume 421, 422 and 423. Alternatively, fluid may be dispensed from the additional volumes only, the fluid in the main volume 311 being used to provide the interior pressure. Fluids are loaded into the volumes 311, 421, 422 and 423 via fittings 34, 451, 452 and 453 respectively. One-way valves are provided in fluid communication with these fittings in the manner described in connection with Fig. 3, and the fluid input may be implemented as discussed in connection with Fig. 3 by techniques such as disclosed in the Quick-Disconnect Valve patent referred to above or by means known in the art. For reasons to be discussed below, the first volume to be filled should be the one intended to be emptied first, the second volume to be filled should be the one intended to be emptied second, and so on; i.e., the volumes should be filled in the order in which they are intended to be emptied.

The fluid conduits (41, 431, 432, 433) from each of volumes 311, 421, 422 and 423 empty through one-way valves 444, 441, 442 and 443 respectively into manifold 46 that goes to the output fitting 37 and output line 371. These one-way valves will not pass fluid in the permitted direction until a specified pressure threshold is reached, and a different threshold is established for each valve. Such pressure-threshold one-way valves are known in the art, and may be implemented, for example, by duck-bill valves. The manifold 46, and the identified fittings and valves are here disposed in member 47, which is contiguous with mandrel 32.

It will be readily apparent that the pressure in volumes 421, 422 and 423 is the same as the pressure in volume 311 . When fluid is permitted to flow through fitting 391, the fluid will flow through the one-way threshold valve 444, 441, 442 or 443 that has the lowest threshold. Assuming that such valve is valve 443, then once flow is established, the pressure from main membrane 31 is exerted to keep this valve open, and flow will continue until volume 423 has emptied completely and flow has ceased. Next, the threshold valve having the next lowest threshold opens, say, valve 442, and then its associated volume 422 empties completely, and so forth. Because of the geometry of the system, it is expected that volume 311 will be the last to empty, and desirably the threshold of its associated valve 444 is the highest.

A system of the general type described in Fig. 4 is applicable, among other things, to systems capable of delivering "SASH," i.e., in sequence, saline, antibiotic, saline and heparin. In order to assure against the possibility that medicine in a secondary volume such as 421, 422 or 423 is not completely emptied as for example by dislocation of its associated membrane, it may in some cases be desirable to establish enough secondary membranes that the main membrane may be loaded with a suitable sterile solution (such as saline) for dispensing last. In this manner the main membrane 31 will force the emptying of all the secondary membranes beneath it. Thus a SASH system would be implemented as a SASH-S system.

Although the multiple volume system of Fig. 4 has been described in connection with a single volume device according to Fig. 3 using a mandrel 32, it could in fact be implemented with prior art single volume systems as well, using secondary volumes placed within the single main volume in a manner analogous to that described in connection with Fig. 4.

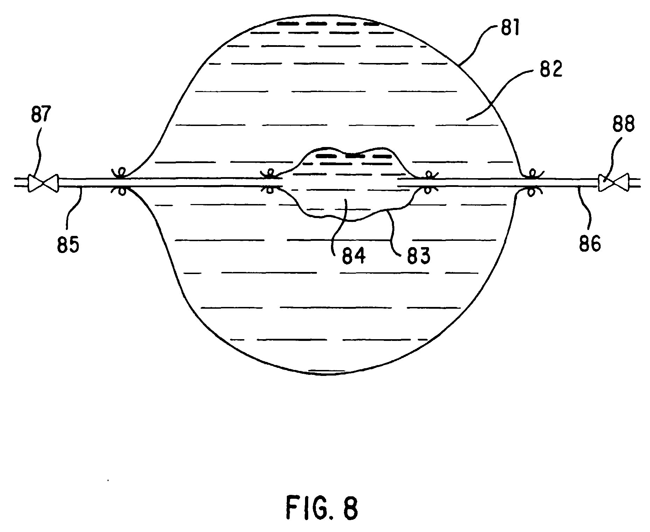

A simple system for delivery of a single fluid at a relatively constant pressure using a pressure chamber having an enclosed pressure fluid is shown in Fig. 8. A resilient membrane 81 encloses a pressure chamber filled with pressure fluid 82 such that membrane 81 is under tension. An additional slack membrane 83, within membrane 81, provides a dispensing chamber of varying dimension which may contain fluid 84, the fluid to be dispensed. A fluid input 85 and a fluid output 86 provide access for fluid 84 to enter and leave the dispensing chamber via valves 87 and 88 respectively. Pressure fluid 82 is sealed within the volume defined by membrane 81 (i.e., the pressure chamber of variable dimension) under pressure such that when the chamber containing fluid 84 is empty, membrane 81 is under tension corresponding to position 12 in Fig. 1. Because membrane 83 is a slack membrane, when the dispensing chamber contains fluid 84 (such as shown in Fig. 8), the pressure of fluid 84 is equal to the pressure of fluid 82. So, when valve 88 is opened, fluid 84, the fluid to be dispensed, is expelled under relatively constant pressure, corresponding to the pressure over range B in Figs. 1 and 2.

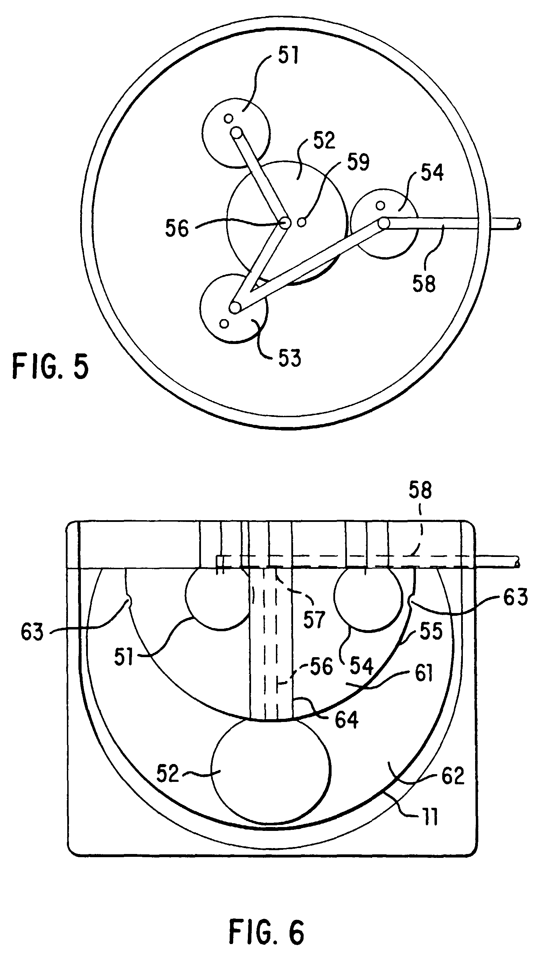

Figs. 5 and 6 show an embodiment similar to the embodiment of Fig. 4 except that fluid is dispensed only from the volumes (or "chambers") defined by additional membranes, not directly from the volume defined by the resilient membrane. Also, in the embodiment of Figs. 5 and 6, three of the additional membranes 51, 53 and 54 are mounted inside volume 61 defined by the mandrel 55. (Additional membrane 52 is mounted between the resilient membrane and the mandrel.) In this embodiment, the pressure chamber includes the volume defined by the mandrel so that the pressure chamber fluid occupies volume 61 defined by the mandrel as well as volume 62 between mandrel 55 and resilient membrane 11. The mandrel has holes 63 in it to allow fluid to pass therethrough so that the pressure of fluid in the pressure chamber is exerted on the additional membranes inside the mandrel. The pressure chamber is filled with fluid and sealed prior to use. The embodiment of Fig. 5, which is 6.4 cm (2.5 inches) in diameter, accepts 24 cc of sterile water (non-dispensed fluid) for use solely to apply pressure to the dispensed fluids in each of the additional volumes. Fig. 5 is a schematic representation of four additional membranes 51, 52, 53 and 54, suitable for accepting SASH fluids. These four membranes are shown again, schematically, in Fig. 6, wherein membrane 53 is hidden directly behind membrane 51. Membrane 52 is shown on the central axis of mandrel 55 and its feed tube 56 extends through mandrel 55 to the spring-loaded valve associated with membrane 52 located in the region 57 and connected to manifold 58. Feed tube 56 and fill tube 59 run parallel to each other through feed post 64 to membrane 52. The fill tube access is a 1.59 mm (1/16 inch) diameter hole.

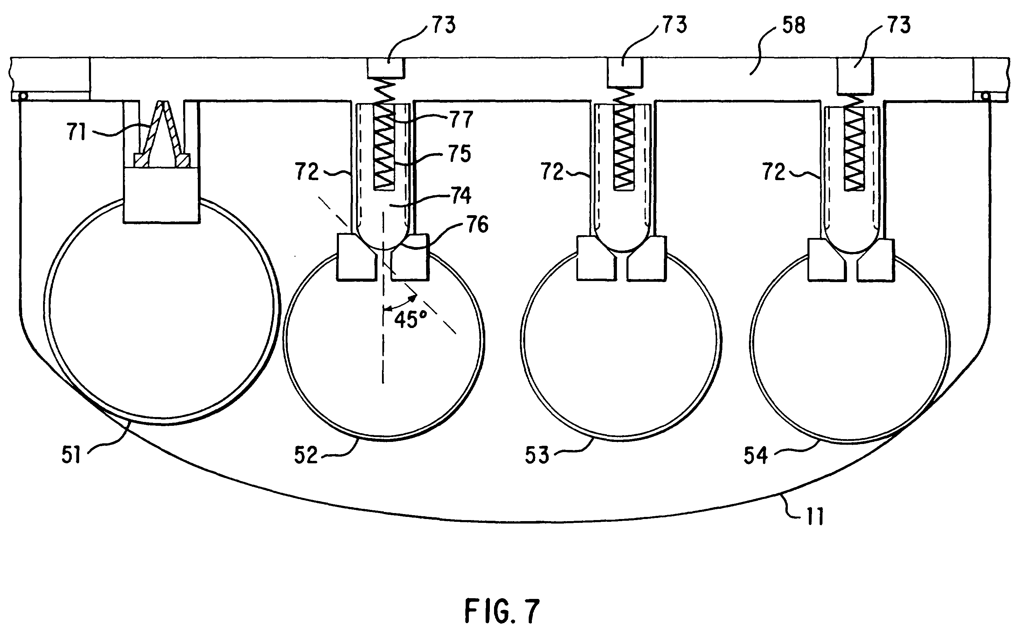

Returning now to Fig. 5, membranes 51, 52, 53 and 54 are seen to be connected in a line along manifold 58. The schematic drawing of Fig. 7 shows the membranes 51-54 enclosed by the resilient membrane 11 and schematically spread out in a line to illustrate the design principles. Membrane 51 is connected to the manifold via duck-bill valve 71. This valve opens first when the line to the patient (e.g., 371 in Fig. 4) is opened. Each of the downstream membranes 52, 53 and 54 is connected to the manifold via a spring-loaded digital valve 72. These valves are identical in construction except for the block 73 which sets the spring-force in its corresponding valve. It can be seen from Fig. 7 that a downstream block is larger than an upstream block. The larger block compresses the spring more, resulting in a greater force to be overcome to open the valve.

Each valve consists of a silicone rubber ball 74, having a spring slot 75, seated against a valve seat 76 and held in place by a spring 77 which is preloaded to the height of a block 73. Except for the blocks, each of which is molded into the cover and has a different height, the components of the valves are identical. Valve seat 76 is chamfered at 45° and the diameter of valve ball 74 is selected so that when it is seated, it defines an area such that the force due to fluid pressure (force = pressure x area) will be just sufficient to overcome the spring force and open the valve. The springs associated with membranes 52, 53 and 54 are preloaded by blocks 73 to provide a force of 184 g (6.5 oz), 377 g (11.9 oz) and 488 g (17.2 oz) respectively. The preload force is set by the height of the block. This wide variation of preload is necessary to ensure proper sequencing in spite of spring tolerances of ±10%.

It has been found beneficial not to have adjacent membranes touch because (i) this can interfere with the equalisation of pressure within the system and (ii) a membrane might be pinched off, preventing it from fully discharging. Thus for a four-fluid (e.g., SASH) system the configuration of Fig. 5 is preferred, having three inner dispensing membranes 51, 53 and 54 and one outer dispensing membrane 52. The three inner dispensing membranes are sized not to touch each other. The outer non-dispensing pressure-producing membrane (resilient membrane 11) does not touch the outer dispensing membrane 52 by virtue of the size of membrane 52 and the amount of fluid in the sealed main volume being sufficient to prevent membrane 11 from contacting mandrel 55 when all dispensing membranes are empty. With dispensing membranes having 2-5 cc capacity, an additional 3 cc of main volume fluid is found to be sufficient.

## Claims

1. A fluid dispensing system comprising:
a resilient membrane (11)(31); and
means for mounting the resilient membrane so as to provide a pressure chamber of variable dimension which constitutes or includes a first dispensable fluid chamber (311)(62) of variable dimension having a fluid conduit (41)(63), the system including
a second dispensable fluid chamber of variable dimension (421), (422), (423), (51), (52), (53), (54) having a fluid conduit (431), (432), (433);
an output manifold (46)(58); and
switch means (441, 442, 443, 444)(71, 72) for serially switching the conduits from the dispensable fluid chambers into the output manifold so as to enable first and second fluids to be sequentially dispensed, characterised in that the second dispensable fluid chamber of variable dimension (421), (422), (423), (51), (52), (53), (54) is disposed within the pressure chamber.

2. A system according to claim 1, wherein the resilient membrane (11)(31) is pre-tensioned beyond the point at which, as dispensable fluid enters the first dispensable fluid chamber and the volume increases, the first derivative of fluid pressure over total fluid volume becomes zero for the first time.

3. A system according to claim 1, further including a pressure fluid disposed in sufficient quantity within the pressure chamber so as to put the resilient membrane under tension.

4. A system according to claim 3, wherein the resilient membrane is pre-tensioned beyond the point at which, as fluid enters the dispensing chamber and the volume of the pressure chamber increases, the first derivative of fluid pressure over total fluid volume becomes zero for the first time.

5. A system according to any preceding claim, wherein the switch means includes a spring-loaded ball valve (72) having a preload exerted by a post (73), the post being sized and positioned such that the preload is in fixed relation to the height of the post.

6. A system according to claim 5, wherein the spring-loaded ball valve includes a ball seat having a chamfered face (76).

7. A system according to any preceding claim, wherein the switch means includes one-way threshold valves (444, 441, 442, 443) between the conduits of the dispensing chambers and the manifold (46)(58).

## Patentansprüche

1. Fluidverteilervorrichtung, umfassend
eine nachgiebige Membran (11, 31); und
Vorrichtungen zur Befestigung der nachgiebigen Membran, so dass eine Druckkammer veränderlicher Größe bereitgestellt ist, welche bildet oder beinhaltet eine erste verteilbare Fluidkammer (311, 62) veränderlicher Größe mit einer Fluidleitung (41, 63), wobei die Vorrichtung beinhaltet
eine zweite verteilbare Fluidkammer veränderlicher Größe (421, 422, 423, 51, 52, 53, 54) mit einer Fluidleitung (431, 432, 433);
einen Auslassverteiler (46, 58); und
Schaltvorrichtungen (441, 442, 443, 444; 71, 72) zum nacheinander Umschalten der Leitungen aus den verteilbaren Fluidkammern in den Auslassverteiler, so dass nacheinander erste und zweite Fluide verteilt werden, dadurch gekennzeichnet, dass die zweite verteilbare Fluidkammer veränderlicher Größe (421, 422, 423; 51, 52, 53, 54) in der Druckkammer angeordnet ist.

2. Vorrichtung nach Anspruch 1, wobei die nachgiebige Membran (11, 31) vorgespannt ist bis zu dem Punkt, an dem die erste Ableitung des Fluiddrucks über das Gesamtfluidvolumen für die erste Zeit Null wird, wenn verteilbares Fluid in die erste verteilbare Fluidkammer eintritt und das Volumen zunimmt.

3. Vorrichtung nach Anspruch 1, zudem beinhaltend ein Druckfluid, das in ausreichender Menge in der Druckkammer angeordnet ist, so dass die nachgiebige Membran unter Spannung gehalten ist.

4. Vorrichtung nach Anspruch 3, wobei die nachgiebige Membran vorgespannt ist bis zu dem Punkt, an dem die erste Ableitung des Fluiddrucks über das Gesamtfluidvolumen für die erste Zeit Null wird, wenn Fluid in die Verteilkammer gelangt und das Volumen der Druckkammer zunimmt.

5. Vorrichtung nach irgendeinem vorhergehenden Anspruch, wobei die Schaltvorrichtung umfasst ein Feder belastetes Kugelventil (72), wobei ein Stab (73) eine Vorlast ausübt, der Stab so bemessen und angeordnet ist, dass die Vorlast in einer festen Beziehung zur Größe des Stabs steht.

6. Vorrichtung nach Anspruch 5, wobei das Feder belastete Kugelventil einen Kugelsitz aufweist, der eine abgeschrägte Seite (76) besitzt.

7. Vorrichtung nach irgendeinem vorhergehenden Anspruch, wobei die Schaltvorrichtung beinhaltet Einwege-Umschlagventile (444, 441, 442, 443) zwischen den Leitungen der Verteilerkammern und des Verteilers (46, 58).

## Revendications

1. Système de distribution de fluide, comprenant :
une membrane élastique (11)(31), et
un dispositif de montage de la membrane élastique afin qu'elle forme une chambre de pression de dimension variable qui constitue ou comprend une première chambre (311)(62) d'un fluide qui peut être distribué, ayant une dimension variable et possédant un conduit de fluide (41)(63), le système comprenant :
une seconde chambre de fluide qui peut être distribué, de dimension variable (421), (422), (423), (51), (52), (53), (54) et ayant un conduit de fluide (431), (432), (433),
un collecteur de sortie (46)(58), et
un dispositif de commutation (441, 442, 443, 444) (71, 72) destiné à commuter en série les conduits des chambres de fluide qui peut être distribué dans le collecteur de sortie pour permettre la distribution successive du premier et du second fluide,
caractérisé en ce que la seconde chambre d'un fluide qui peut être distribué et ayant une dimension variable (421), (422), (423), (51), (52), (53), (54) est disposée dans la chambre de pression.

2. Système selon la revendication 1, dans lequel la membrane élastique (11)(31) subit une tension préalable au-delà du point auquel, lorsqu'un fluide qui peut être distribué pénètre dans la première chambre de fluide qui peut être distribué et le volume augmente, la dérivée première de la pression du fluide dans tout le volume de fluide devient nulle pour la première fois.

3. Système selon la revendication 1, comprenant en outre un fluide sous pression placé en quantité suffisante dans la chambre de pression pour placer la membrane élastique sous tension.

4. Système selon la revendication 3, dans lequel la membrane élastique subit une tension préalable au-delà du point auquel, lorsque le fluide pénètre dans la chambre de distribution et le volume de la chambre de pression augmente, la dérivée première de la pression du fluide dans tout le volume de fluide devient nulle pour la première fois.

5. Système selon l'une quelconque des revendications précédentes, dans lequel le dispositif de commutation comprend un clapet à bille (72) rappelé par un ressort et ayant une charge préalable appliquée par un plot (73), le plot ayant une dimension et une disposition telles que la charge préalable présente une relation fixe avec la hauteur du plot.

6. Système selon la revendication 5, dans lequel le clapet à bille rappelé par un ressort comprend un siège de bille ayant une face chanfreinée (76).

7. Système selon l'une quelconque des revendications précédentes, dans lequel le dispositif de commutation comprend des clapets à seuil (444, 441, 442, 443) placés entre les conduits des chambres de distribution et le collecteur (46)(58).
